# EUROPÄISCHE PATENTSCHRIFT

(11) **EP 1 325 458 B1**
(45) Veröffentlichungstag und Bekanntmachung des Hinweises auf die Patenterteilung: **21.11.2007**
(21) Anmeldenummer: 01980335.2
(22) Anmeldetag: 05.09.2001
(51) Int. Cl.: G06F 19/00

(54) **SYSTEM UND VERFAHREN ZUR ZENTRALEN STEUERUNG VON EINRICHTUNGEN, DIE WÄHREND EINER OPERATION BENUTZT WERDEN**
SYSTEM AND METHOD FOR THE CENTRAL CONTROL OF DEVICES USED DURING AN OPERATION
SYSTEME ET PROCEDE DE COMMANDE CENTRALE DE DISPOSITIFS UTILISES LORS D'UNE OPERATION

(30) Priorität: 05.09.2000 EP 00119179
(43) Veröffentlichungstag der Anmeldung: 09.07.2003
(73) Patentinhaber: Storz-Endoskop GmbH, 8200 Schaffhausen (CH)
(72) Erfinder: STILLER, Heinz-Werner, CH-8222 Beringen (CH); NOVAK, Pavel, CH-8207 Schaffhausen (CH); IRION, Klaus, M., 78576 Liptingen (DE)
(74) Vertreter: Lindner, Michael
(86) Internationale Anmeldenummer: PCT/EP2001/010189
(87) Internationale Veröffentlichungsnummer: WO 2002/019957

(56) Entgegenhaltungen:
- EP-A- 0 747 279
- WO-A-99/21165
- US-A- 5 572 999
- US-A- 5 788 688
- US-A- 5 819 229

## Beschreibung

Die vorliegende Erfindung betrifft ein System und ein Verfahren zur zentralen Steuerung von Einrichtungen, insbesondere medizinischen Geräten, die während einer Operation benutzt werden, mit einer ersten Steuerungseinheit zur Steuerung der Einrichtungen.

Aus ergonomischen Gründen ist es wünschenswert, die Steuerung aller während einer Operation benötigten Systeme von einer zentralen Stelle, möglichst aus dem sterilen Bereich, fernsteuern bzw. bedienen zu können. Eine solche Steuerung kann z.B. durch ein Touch-Screen (mit sterilem Überzug) oder mit einer Sprachsteuerung erfolgen. Zu den gesteuerten Einrichtungen bzw. Systemen können bspw. endoskopische Geräte gehören, sowie OP-Tisch, OP-Beleuchtung, Raumbeleuchtung, Klimaanlage, Telefon, Pager, Internet, Krankenhaus-Informationssystem, Verbrauchsmaterial, Verwaltungssystem und andere.

Aus WO 99/21165 A1 ist ein System zur Steuerung von Geräten in einem Operationssaal bekannt. Zur Steuerung von medizinischen Geräten ist in der DE 199 04 090 A1 bspw. angegeben, die einzelnen Geräte über einen CAN-Bus miteinander zu verbinden, wobei die einzelnen Geräte als Slaves und ein Leitrechner als Master dienen. Über diesen Leitrechner sind alle Geräte steuerbar.

Der Nachteil einer solchen Vernetzung ist insbesondere darin zu sehen, daß der Software- und Hardware-Aufwand für den einzigen Leitrechner sehr hoch ist, da er an das zu steuernde Gerät mit den höchsten Sicherheitsanforderungen angepaßt sein muß. Bei zu steuernden Geräten, die nicht diese hohen Sicherheitsanforderungen erfüllen müssen, geht dadurch möglicherweise Flexibilität oder die Einfachheit der Bedienung verloren.

Wird der einzige Leitrechner im Hinblick auf Sicherheitsaspekte nach weniger strengen Maßstäben aufgebaut, so bestünde jedoch die Gefahr (z.B. bei einem PC mit Standard-Software wie Windows-NT), daß sicherheitsrelevante Funktionen durch unzuverlässige Funktionen der nicht-sicherheitsrelevanten Systeme gefährdet würden.

Hierbei wird davon ausgegangen, daß die genannten medizinischen Geräte in zwei unterschiedliche Gruppen eingeteilt werden können, nämlich einerseits sicherheitsrelevante Systeme wie z.B. endoskopische Geräte (Insufflation, Pumpen, HF-Chirurgie usw.), OP-Tisch-Steuerung etc., also Geräte oder Systeme, die bei Ausfall oder Fehler für den Patienten lebensbedrohlich sein können, und andererseits nicht-sicherheitsrelevante Systeme, wie Bildarchivierung, Materialverwaltungssysteme, Telefon-fernbedienung usw.

Vor diesem Hintergrund besteht die Aufgabe der vorliegenden Erfindung darin, ein System bzw. ein Verfahren anzugeben, dem die vorgenannten Nachteile nicht mehr anhaften. Insbesondere soll es die Sicherheit bezüglich der Steuerung sicherheitsrelevanter Geräte erhöhen.

Diese Aufgabe wird bei dem System der eingangs genannten Art durch die Merkmale des Anspruchs 1 gelöst.

Das heißt mit anderen Worten, daß das erfindungsgemäße System nicht mehr wie bisher einen einzigen Leitrechner, sondern statt dessen zwei Steuerungseinheiten einsetzt, die jeweils unterschiedlichen Gruppen von zu steuernden Geräten zugeordnet sind. Bei der Zuordnung der zu steuernden Einrichtungen zu den beiden Steuerungseinheiten wird davon ausgegangen, daß es grundsätzlich bei einer Operation Einrichtungen zur Ausführung sicherheitsrelevanter Funktionen (bspw. endoskopische Geräte) und andererseits Einrichtungen zur Ausführung nicht-sicherheitsrelevanter Funktionen, wie bspw. Raumbeleuchtung, Klimaanlage etc., gibt.

In diesem Zusammenhang ist unter dem Begriff "geschlossenes System" ein System zu verstehen, das keine Einflußnahme von außen erlaubt. Ein solches System kann weder direkt durch den Benutzer noch über Internet etc. manipuliert, neu konfiguriert etc. werden. Im Gegensatz dazu kann ein offenes System vom Benutzer konfiguriert oder bspw. ergänzt werden. Hier sind also Eingriffe bzw. Manipulationen von außen möglich.

Der Vorteil des erfindungsgemäßen Systems ist u.a. darin zu sehen, daß das Vorsehen einer weiteren Steuerungseinheit die Sicherheit gegenüber ungewollten Fehlfunktionen erhöht, ohne die Flexibilität des Gesamtsystems hierdurch zu beschränken. Dadurch, daß bspw. auf der zweiten Steuerungseinheit Software zum Einsatz kommen kann, die nicht die strengen Sicherheitskriterien zur Steuerung sicherheitsrelevanter Einrichtungen erfüllen muß, kann auf Standard-Software zurückgegriffen werden, so daß einerseits die einmaligen Investitionskosten als auch die laufenden Wartungskosten des Gesamtsystems reduziert werden.

Ein weiterer Vorteil des erfindungsgemäßen Systems kann darin gesehen werden, daß die erste Steuerungseinheit, die für die Steuerung der sicherheitsrelevanten Einrichtungen zuständig ist, als geschlossenes System ausgebildet ist, bei dem sichergestellt ist, daß sämtliche Zugriffe zur Manipulation des Betriebssystems gesperrt sind. Darüber hinaus sind auch Manipulationen an den Applikationen zur Steuerung der sicherheitsrelevanten Einrichtungen unmöglich.

An dieser Stelle sei jedoch angemerkt, daß auf der ersten Steuerungseinheit auch Applikationen zur Steuerung nicht-sicherheitsrelevanter Einrichtungen ablaufen können, sofern diese Applikationen zuvor unter Sicherheitsaspekten getestet wurden.

Es ist eine Schnittstelleneinheit vorgesehen, die einerseits mit den beiden Steuerungseinheiten und andererseits mit Peripheriegeräten verbunden ist und jeweils eine der beiden Steuerungseinheiten mit den Peripheriegeräten verbindet. Bevorzugt wird die Schnittstelleneinheit über eine Steuerleitung von der ersten Steuerungseinheit gesteuert. Weiter bevorzugt zählen zu den Peripheriegeräten eine Anzeigeeinrichtung und/oder eine Eingabeeinrichtung, vorzugsweise eine Tastatur und eine Maus. Weiter bevorzugt ist die Anzeigeeinrichtung als Touch-Screen ausgebildet, so daß auch Eingaben darüber möglich sind.

Diese vorgenannten Maßnahmen führen zu dem Vorteil, daß einerseits die Kosten für das Gesamtsystem reduziert werden und andererseits die Bedienbarkeit deutlich vereinfacht wird, da die zur Eingabe von Steuerungsbefehlen oder zur Überwachung von Parametern erforderlichen. Peripheriegeräte nur einmal vorhanden sein müssen. Der Operateur muß folglich nicht mehrere Anzeigeeinrichtungen im Blick haben. Ferner gewährleistet die Steuerleitung zwischen Schnittstelleneinheit und erster Steuerungseinheit, daß die die wichtigen sicherheitsrelevanten Einrichtungen steuernde Steuerungseinheit auch im Falle eines Ausfalls bzw. eines Fehlers der zweiten Steuerungseinheit die entsprechenden erforderlichen Funktionen zuläßt und die wichtigen sicherheitsrelevanten Parameter auf dem Touch-Screen darstellt. Insgesamt wird also eine Erhöhung der Sicherheit des Systems erreicht.

Die zweite Steuerungseinheit weist ein Empfangsmittel auf, um Fehlermeldungen von der ersten Steuerungseinheit zu erfassen und auf einem der Peripheriegeräte darzustellen.

Diese Maßnahme hat den Vorteil, daß auch im Falle einer Verbindung der zweiten Steuerungseinheit zur Steuerung nicht sicherheitsrelevanter Einrichtungen mit der Schnittstelleneinheit Fehlermeldungen bezüglich sicherheitsrelevanter Einrichtungen dem Benutzer des Systems sofort übermittelt werden. Damit läßt sich vermeiden, daß die Darstellung solcher Fehlermeldungen erst beim erneuten Umschalten der Verbindung von erster Steuerungseinheit zu Peripheriegeräten dem Benutzer gemeldet wird. Dies hat folglich den Vorteil, daß die Sicherheit des Gesamtsystems weiter erhöht wird.

Vorteilhafterweise sind die beiden Steuerungseinheiten jeweils Bestandteil eigenständiger Rechner (PC). Selbstverständlich ist es auch denkbar, die beiden Steuerungseinheiten in einen Rechner zu integrieren, der über zumindest zwei Prozessoren (CPU) verfügt, wobei jeweils eine Steuerungseinheit von einem Prozessor realisiert wird.

Bei einem Einsatz eines einzigen Leitrechners wäre es wie bisher nicht möglich, bei derartigen Vorgaben die Steuerung der nicht-sicherheitsrelevanten Einrichtungen mit der gewünschten Einfachheit und Flexibilität auszuführen, wobei zudem immer die Gefahr bestünde, daß fehlerhaft programmierte Software für die nicht-sicherheitsrelevanten Einrichtungen Einfluß nimmt auf die Steuerung der sicherheitsrelevanten Einrichtungen.

In einer bevorzugten Weiterbildung sind die erste Steuerungseinheit und die sicherheitsrelevanten Einrichtungen über ein BUS-System, vorzugsweise den Karl Storz-Communication-BUS (SCB®), miteinander verbunden. Vorzugsweise sind die nicht-sicherheitsrelevanten Einrichtungen und die zweite Steuerungseinheit über ein weiteres BUS-System miteinander verbunden, wobei die beiden BUS-Systeme vorzugsweise unterschiedlich sind.

Diese Maßnahmen führen zu einer Vereinfachung des Systems sowie zu einer Reduzierung der Gesarntkosten, da das besonders ausgebildete BUS-System für sicherheitskritische Funktionen nicht zur Steuerung jeder Einrichtung zum Einsatz kommt. Vielmehr kann auch auf gängige Standard-BUS-Systeme zurückgegriffen werden. Das aufwendige sichere BUS-System wird also nur für die Verbindung mit den sicherheitsrelevanten Einrichtungen eingesetzt.

Vorzugsweise gehören zu den sicherheitsrelevanten Einrichtungen endoskopische Geräte, vorzugsweise Insufflatoren, Pumpen, Lichtquellen, Videogeräte und bspw. OP-Tisch-Steuerung usw. Zu den nicht-sicherheitsrelevanten Einrichtungen gehören bspw. Bildarchivierung, OP-Beleuchtung, Raumbeleuchtung, Telefon, Klimaanlage, Pager, Internet, Krankenhaussystem, Verbrauchsmaterial, Verwaltungssysteme etc.

Es ist weiter bevorzugt, die beiden Steuerungseinheiten über einen Ethernet-BUS (TCP/IP-Protokoll) miteinander zu verbinden, da sich dieses BUS-System als zuverlässiges, kostengünstiges System herausgestellt hat.

Die erste Steuerungseinheit weist ein eingebettetes Betriebssystem, vorzugsweise "embedded Windows NT", auf, das gegenüber Eingriffen von außen geschützt ist.

Dies heißt mit anderen Worten, daß das Betriebssystem der ersten Steuerungseinheit fester Bestandteil der Einheit ist und damit gegenüber Manipulationen geschützt ist. Der Benutzer kann keine Eingriffe in das Betriebssystem vornehmen, wie dies bspw. bei gängigen PCs möglich wäre. Damit wird vermieden, daß durch beabsichtigte oder unbeabsichtigte Eingriffe in das Betriebssystem bestimmte sicherheitsrelevante Funktionen nicht mehr oder fehlerhaft ausgeführt werden.

In einer bevorzugten Weiterbildung weist die erste Steuerungseinheit ein Prüfmittel auf, das die Verbindung zu der Schnittstelleneinheit zyklisch prüft und eine Fehlermeldung abgibt, wenn eine Verbindung nicht vorhanden ist.

Auch diese Maßnahme führt insgesamt zu einer Steigerung der Sicherheit, da das System dem Benutzer sofort mitteilen kann, wenn eine Darstellung bzw. eine Einstellung bestimmter Parameter sicherheitsrelevanter Einrichtungen aufgrund einer fehlerhaften Schnittstelleneinheit nicht mehr möglich ist.

In einer bevorzugten Weiterbildung umfaßt die erste Steuerungseinheit eine Sprachsteuerungseinheit, bspw. in Form eines Softwaremoduls.

Diese Maßnahme hat den Vorteil, daß sich die Bedienung für den Operateur vereinfacht.

Die der Erfindung zugrunde liegende Aufgabe wird auch von einem Verfahren zur zentralen Steuerung von Einrichtungen, die während einer Operation benutzt werden, durch die Merkmale des Anspruchs 15 gelöst.

Dieses Verfahren ermöglicht die Verwirklichung der bereits im Zusammenhang mit dem erfindungsgsmäßen System genannten Vorteile in gleicher Weise, so daß die Vorteile an dieser Stelle nicht nochmals erwähnt werden müssen.

Die beiden Steuerungseinheiten kommunizieren miteinander, wobei insbesondere die erste Steuerungseinheit die zweite Steuerungseinheit auf Fehler überprüft. Es wird eine Schnittstelleneinheit vorgesehen, die von der ersten Steuernungseinheit gesteuert wird und abhängig davon Signale entweder von der ersten oder der zweiten Steuerungseinheit an gemeinsame Peripheriegeräte weiterleitet.

Diese Maßnahme hat - wie bereits erwähnt - den Vorteil, die Kosten des Systems zu reduzieren und die Übersichtlichkeit der Bedienung und den Bedienungskomfort insgesamt zu steigern.

Die erste Steuerungseinheit wird bei einem Fehler der zweiten Steuerungseinheit die Schnittstelleneinheit so ansteuern, daß die Signale der ersten Steuerungseinheit an die Peripheriegeräte weitergeleitet werden.

Mit anderen Worten heißt das, daß die erste Steuerungseinheit gewährleistet, daß ein Fehler in der zweiten Steuerungseinheit nicht zu einem Ausfall der Verbindung von erster Steuerungseinheit zu Peripheriegeräten führen kann.

In einer bevorzugten Weiterbildung leitet die Schnittstelleneinheit die Signale der ersten Steuerungseinheit an die Peripheriegeräte sofort weiter, wenn eine sicherheitsrelevante Funktion ausgeführt werden soll.

Diese Maßnahme hat den Vorteil, daß die wichtigen Funktionen auch bei zunächst vorhandener Verbindung zwischen der zweiten Steuerungseinheit und den Peripheriegeräten möglich ist. Eine Erhöhung der Sicherheit ist die Folge.

In einer bevorzugten Weiterbildung leitet die Schnittstelleneinheit nach dem Aktivieren einer nicht-sicherheitsrelevanten Funktion die Signale der zweiten Steuerungseinheit an die Peripheriegeräte erst dann weiter, wenn die sicherheitsrelevante Funktion vollständig ausgeführt bzw. abgearbeitet ist. Das heißt mit anderen Worten, daß die Ausführung sicherheitsrelevanter Funktionen nicht durch Umschalten der Schnittstelleneinheit unterbrochen werden kann. Vielmehr wird die Ausführung der sicherheitsrelevanten Funktion bis zum Ende ausgeführt, und erst dann wird die Schnittstelleneinheit die Verbindung zwischen zweiter Steuerungseinheit und Peripheriegeräten herstellen.

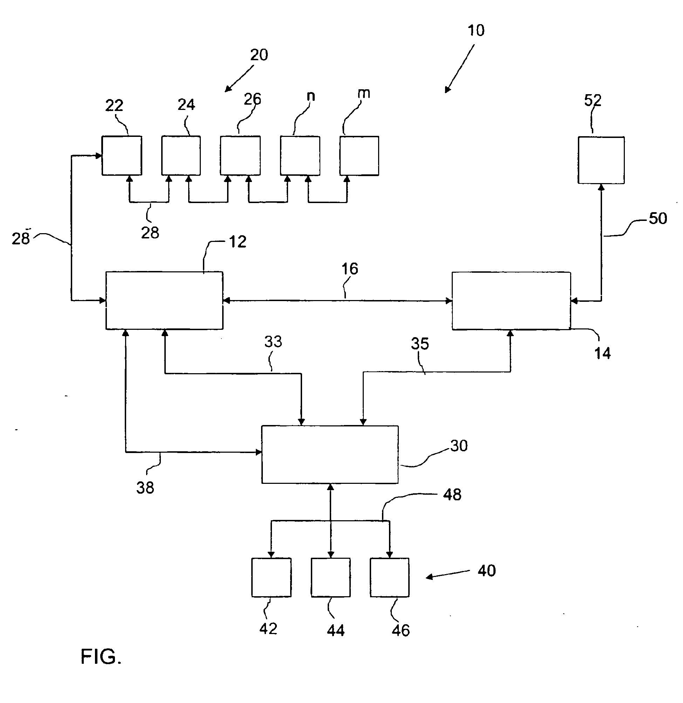

Weitere Vorteile und Ausgestaltungen der Erfindung ergeben sich aus der Beschreibung und der beiliegenden Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nun anhand einer Figur näher beschrieben, wobei die Figur ein schematisches Blockschaltdiagramm eines erfindungsgemäßen Systems darstellt.

In der Figur ist ein System zur zentralen Steuerung von Einrichtungen, die während einer Operation benutzt werden, in Form eines Blockschaltdiagramms dargestellt und mit dem Bezugszeichen 10 gekennzeichnet. Das System 10 umfaßt eine erste Rechnereinheit 12 und eine zweite Rechnereinheit 14. Beide Rechnereinheiten 12, 14 sind über eine BUS-Verbindung 16, bspw. eine Ethernet-BUS-Verbindung, miteinander verbunden, um Daten in Form von Nachrichten auszutauschen.

Beide Rechnereinheiten 12, 14 sind als medizinische PCs ausgebildet, wobei in der ersten Rechnereinheit 12 ein eingebettetes Betriebssystem, vorzugsweise ein "embedded Windows NT"-Betriebssystem, zum Einsatz kommt. Die zweite Rechnereinheit 14 arbeitet vorzugsweise mit einem üblichen Windows oder einem anderen nicht-eingebetteten Betriebssystem.

Die erste Rechnereinheit 12 dient zumindest zur Steuerung von medizinischen Einrichtungen, die sicherheitsrelevante bzw. sicherheitskritische Funktionen ausführen. In der Figur sind diese sicherheitsrelevanten Geräte mit dem Bezugszeichen 20 gekennzeichnet. Beispielhaft sind in der Figur eine Pumpe 22, ein Insufflator 24 und ein HF-Generator 26 gezeigt. Diese beispielhafte Aufzählung dreier Geräte ist jedoch nicht einschränkend zu verstehen, was in der Figur durch weitere Geräte n und m angedeutet wird. Darüber hinaus kann die erste Rechnereinheit 12 auch zur Steuerung nicht-sicherheitsrelevanter Geräte bei Verwendung entsprechend getesteter Software eingesetzt werden. Im folgenden soll jedoch auf diese Möglichkeit jedoch nicht weiter eingegangen werden.

Die Kommunikation zwischen der ersten Rechnereinheit 12 und den sicherheitsrelevanten Geräten 20 erfolgt über ein BUS-System 28, das eine sichere Übertragung von Daten ermöglicht. Bei diesem BUS-System 28 sind andere Kriterien bezüglich Fehlersicherheit als bspw. bei dem vorgenannten Ethernet-BUS 16 anzulegen. Die Anmelderin bietet ein solches BUS-System bspw. unter dem Namen Karl Storz-Communication-BUS (SCB®) an.

Das System 10 umfaßt ferner eine Umschalteinheit 30. Die Umschalteinheit 30 ist eingangsseitig mit der Rechnereinheit 12 und der Rechnereinheit 14 verbunden, wobei in der Figur beispielhaft nur jeweils eine Verbindungsleitung 33, 35 dargestellt ist. Es versteht sich, daß es sich bei den beiden Verbindungen 33, 35 um eine Vielzahl von einzelnen Verbindungsleitungen handelt.

Ausgangsseitig ist die Umschalteinheit 30 mit Peripheriegeräten 40 verbunden, wobei stellvertretend in der Figur ein berührungsempfindlicher Monitor 42 (Touch-Screen genannt), eine Eingabetastatur 44 sowie eine Maus 46 gezeigt sind. Die Peripheriegeräte 40 befinden sich bspw. im direkten Umfeld eines Operateurs im Operationssaal, so daß diese Peripheriegeräte 40 entsprechend angepaßt sein müssen. Der Touch-Screen 42 ist bspw. mit einem sterilen Überzug versehen.

Die Verbindung der Peripheriegeräte 40 mit der Umschalteinheit 30 erfolgt über entsprechende Leitungen 48, wobei auch hier der Einfachheit wegen nur jeweils eine Leitung stellvertretend für eine Vielzahl von Verbindungsleitungen dargestellt ist.

Die Umschalteinheit 30 hat nun die Aufgabe, die einzelnen Peripheriegeräte 42 bis 46 mit einer der beiden Rechnereinheiten 12, 14 zu verbinden, so daß die Eingabe bzw. die Darstellung von Daten möglich wird.

Die Steuerung der Umschalteinheit 30 übernimmt die erste Rechnereinheit 12, wobei über eine Steuerleitung 38 entsprechende Steuersignale an die Umschalteinheit 30 übermittelt werden können.

Die zweite Rechnereinheit 14 ist über einen optischen BUS 50 mit Geräten 52 (galvanisch getrennt) verbunden, die nichtsicherheitsrelevante Funktionen ausführen. Hierzu gehören bspw. eine Telefon-Fernbedienung, die Raumbeleuchtung etc. Die Steuerung dieser nicht-sicherheitsrelevanten Geräte wird somit von der zweiten Rechnereinheit 14 geleistet.

Wie bereits erwähnt, ist die erste Rechnereinheit 12 mit einem eingebetteten Betriebssystem ausgestattet. Dies soll gewährleisten, daß Eingriffe bzw. Manipulationen am System von außen nicht möglich sind. Die erste Rechnereinheit 12 ist vielmehr als geschlossenes System ausgebildet, auf dem im wesentlichen nur Tasks ablaufen, die für die Steuerung der sicherheitsrelevanten Geräte 20 erforderlich sind. Bei entsprechender getesteter Eignung können daneben auch Tasks ablaufen, die der Steuerung der nicht-sicherheitsrelevanten Geräte 52 dienen.

Die zweite Rechnereinheit 14 ist dagegen als üblicher medizinischer PC ausgebildet. Im Gegensatz zu der ersten Rechnereinheit 12 können auf der zweiten Rechnereinheit 14 keine Tasks ablaufen, die der Steuerung sicherheitsrelevanter Geräte dienen.

Beide Rechnereinheiten 12, 14 liefern Daten über die entsprechenden Leitungen 33, 35 an die Umschalteinheit 30, wobei je nach "Stellung" der Umschalteinheit nur die Daten einer der beiden Rechnereinheiten auf dem Touch-Screen 42 dargestellt werden. Auch die Eingabe von Daten erfolgt dann nur in diese Rechnereinheit. Möchte der Operateur bspw. Funktionen der anderen Gruppe von Geräten auswählen, kann er dies über entsprechende Eingabe eines Befehls, der entweder direkt von der ersten Rechnereinheit 12 oder indirekt über die Rechnereinheit 14 und den BUS 16 von der ersten Rechnereinheit 12 empfangen wird. Diese sendet daraufhin ein entsprechendes Steuersignal über die Steuerleitung 38 aus, was ein Umschalten in der Umschalteinheit 30 zur Folge hat. Auf dem Touch-Screen 42 werden anschließend die entsprechenden Daten, Auswahlmenüs etc. der ausgewählten Gruppe von Geräten dargestellt.

Im Falle einer Verbindung der zweiten Rechnereinheit 14 mit den Peripheriegeräten 40 ist es jedoch erforderlich, daß jegliche Fehlermeldungen, die sicherheitsrelevante Geräte 20 betreffen, sofort dem Operateur unabhängig von dem Schaltzustand der Umschalteinheit 30 über.den Touch-Screen 42 mitgeteilt werden. In der zweiten Rechnereinheit 14 läuft hierfür eine Task, die ständig die über den BUS 16 von der ersten Rechnereinheit 12 gelieferten Nachrichten auf Fehlermeldungen überprüft. Bei Feststellen einer Fehlermeldung sorgt die zweite Rechnereinheit 14 dafür, daß auf dem Touch-Screen ein Fenster geöffnet wird, in dem diese Fehlermeldung dargestellt wird.

Eine weitere Aufgabe der ersten Rechnereinheit 12 besteht darin, das Vorhandensein der Umschalteinheit 30 zu überprüfen. Sollte die Umschalteinheit 30 bspw. durch Ausfall für die erste Rechnereinheit 12 nicht mehr zu erkennen sein, muß sie sofort eine Fehlermeldung abgeben. Diese Fehlermeldung soll dem Operateur signalisieren, daß eine richtige Darstellung und eine Eingabe von Daten über die Peripheriegeräte 40 nicht mehr gewährleistet ist.

Ferner ist es notwendig, daß die erste Rechnereinheit 12 die zweite Rechnereinheit 14 überprüft und im Fehlerfall die Umschalteinheit 30 sofort in denjenigen Schaltzustand steuert, bei dem die erste Rechnereinheit 12 mit den Peripheriegeräten 40 verbunden ist.

Unter Sicherheitsaspekten ist es zudem erforderlich, daß bei der Eingabe eines Befehls zur Umschaltung der Peripheriegeräte 40 auf die zweite Rechnereinheit 14 zunächst alle noch nicht beendeten Funktionen der sicherheitsrelevanten Geräte 20 ausgeführt werden mit einer entsprechenden Darstellung der Parameter. Dies soll gewährleisten, daß die Ausführung dieser sicherheitsrelevanten Funktionen nicht zu früh beendet wird. Im umgekehrten Fall werden die Peripheriegeräte 40 jedoch sofort mit der ersten Rechnereinheit 12 verbunden, so daß sicherheitsrelevante Funktionen ohne Wartezeit sofort ausgeführt werden können.

Es versteht sich, daß die Erfindung nicht nur in der zuvor beschriebenen Ausführungsform, sondern auch in anderen Ausführungsformen realisiert werden kann. Der Umfang solcher Änderungen wird alleine durch die angehängten Ansprüche definiert.

## Patentansprüche

1. System zur zentralen Steuerung von Einrichtungen (20, 52), die während einer Operation benutzt werden, mit einer ersten Steuerungseinheit (12) zur Steuerung der Einrichtungen, **dadurch gekennzeichnet, daß** eine zweite Steuerungseinheit (14) vorgesehen ist, die mit der ersten Steuerungseinheit (12) zum Austausch von Nachrichten verbunden ist, und daß die erste Steuerungseinheit (12) als geschlossenes System mit einem eingebetteten Betriebssystem ausgestattet ist und zur Steuerung zumindest derjenigen Einrichtungen (20; 22-26) ausgebildet ist, die sicherheitsrelevante Funktionen ausführen, daß die erste Steuerungseinheit (12) die zweite Steuerungseinheit (14) überprüft, und daß die zweite Steuerungseinheit (14) als offenes System mit einem nicht-eingebetteten Betriebssystem ausgestattet ist und zur Steuerung der übrigen Einrichtungen (52) ausgebildet ist, die nicht sicherheitsrelevante Funktionen ausführen, und daß die zweite Steuereinheit (14) die von der ersten Steuerungseinheit (12) gelieferten Nachrichten auf Fehlermeldungen überprüft und bei einer Fehlermeldung diese auf einem Peripheriegerät darstellt, und daß eine Schnittstelleneinheit (30) vorgesehen ist, die einerseits mit den beiden Steuerungseinheiten (12, 14) und andererseits mit Peripheriegeräten (40) verbunden ist und Jeweils eine der beiden Steuerungseinheiten (12, 14) mit den Peripheriegeräten (40) verbindet.

2. System nach Anspruch 1, **dadurch gekennzeichnet, daß** die erste Steuerungseinheit (12) und die sicherheitsrelevanten Einrichtungen (20) über ein Bussystem (28) miteinander verbunden sind.

3. System nach Anspruch 1 oder 2, **dadurch gekennzeichnet, daß** die nicht-sicherheitsrelevanten Einrichtungen (52) und die zweite Steuerungseinheit (14) über ein weiteres Bussystem (50) miteinander verbunden sind.

4. System nach Anspruch 2 und 3, **dadurch gekennzeichnet, daß** die beiden Bussysteme (28, 50) unterschiedlich sind.

5. System nach Anspruch 1, **dadurch gekennzeichnet, daß** die Schnittstelleneinheit (30) über eine Steuerleitung (38) von der ersten Steuerungseinheit (12) gesteuert ist.

6. System nach Anspruch 1 oder 5, **dadurch gekennzeichnet, daß** zu den Peripheriegeräten (40) eine Anzeigeeinrichtung (42) und/oder eine Eingabeeinrichtung (44, 46), vorzugsweise eine Tastatur und eine Maus, zählt.

7. System nach Anspruch 6, **dadurch gekennzeichnet, daß** die Anzeigeeinrichtung (42) als Touch-Screen ausgebildet ist, so daß auch Eingaben darüber möglich sind.

8. System nach Anspruch 1, 5, 6 oder 7, **dadurch gekennzeichnet, daß** die zweite Steuerungseinheit (14) ein Empfangsmittel aufweist, um Fehlermeldungen von der ersten Steuerungseinheit (12) zu erfassen und auf einem der Peripheriegeräte (40) darzustellen.

9. System nach einem der vorhergehenden Ansprüche, **dadurch gekennzeichnet, daß** die sicherheitsrelevanten Einrichtungen (20) endoskopische Geräte, vorzugsweise Insufflatoren, Pumpen, Lichtquellen, Videogeräte usw, umfassen.

10. System nach einem der vorhergehenden Ansprüche, **dadurch gekennzeichnet, daß** die nicht-sicherheitsrelevanten Einrichtungen (52) Bildarchivierung, OP-Beleuchtung, Raumbeleuchtung, Telefon, Klimaanlage, Pager, Internet, Krankenhaussystem, Verbrauchsmaterial, Verwaltungssysteme etc. umfassen können.

11. System nach einem der vorhergehenden Ansprüche, **dadurch gekennzeichnet, daß** die beiden Steuerungseinheiten (12, 14) über einen Ethernet-Bus (16) miteinander verbunden sind.

12. System nach einem der vorhergehenden Ansprüche, **dadurch gekennzeichnet, daß** die erste Steuerungseinheit (12) eine Sprachsteuerungseinheit aufweist.

13. System nach einem der vorhergehenden Ansprüche, **dadurch gekennzeichnet, daß** die beiden Steuerungseinheiten (12, 14) als eine Rechnereinheit mit zwei Prozessoren (CPU) ausgebildet sind.

14. System nach Anspruch 1, **dadurch gekennzeichnet, daß** die erste Steuerungseinheit ein Prüfmittel aufweist, das die Verbindung zu der Schnittstelleneinheit (30) zyklisch prüft und eine Fehlermeldung abgibt, wenn eine Verbindung nicht vorhanden ist.

15. Verfahren zur zentralen Steuerung von Einrichtungen, die während einer Operation benutzt werden, **dadurch gekennzeichnet, daß**
die Einrichtungen zur Ausführung sicherheitsrelevanter Funktionen von einer ersten Steuerungseinheit (12) als geschlossenes System mit eingebettetem Betriebssystem und die Einrichtungen zur Ausführung nicht-sicherheitsrelevanter Funktionen von einer zweiten Steuerungseinheit (14) als offenes System mit nicht-eingebettetem Betriebssystem gesteuert werden,
die beiden Steuerungseinheiten (12, 14) miteinander kommunizieren, wobei insbesondere die erste Steuerungseinheit die zweite Steuerungseinheit auf Fehler überprüft,
eine Schnittstelleneinheit (30) bereitgestellt wird, die von der ersten Steuerungseinheit (12) gesteuert wird und abhängig davon Signale entweder von der ersten oder der zweiten Steuerungseinheit an gemeinsame Peripheriegeräte (40) weiterleitet, und
die erste Steuerungseinheit (12) bei einem Fehler der zweiten Steuerungseinheit (14) die Schnittstelleneinheit (30) so ansteuert, daß die Signale der ersten Steuerungseinheit (12) an die Peripheriegeräte (40) weitergeleitet werden.

16. Verfahren nach Anspruch 15, **dadurch gekennzeichnet, daß** die Schnittstelleneinheit (30) die Signale der ersten Steuerungseinheit (12) an die Peripheriegeräte (40) sofort weiterleitet, wenn eine sicherheitsrelevante Funktion ausgeführt werden soll.

17. Verfahren nach Anspruch 15, **dadurch gekennzeichnet, daß** nach dem Aktivieren einer nicht-sicherheitsrelevanten Funktion die Schnittstelleneinheit (30) die Signale der zweiten Steuerungseinheit (14) an die Peripheriegeräte (40) erst dann weiterleitet, wenn die sicherheitsrelevante Funktion vollständig ausgeführt ist.

## Claims

1. System for the central control of devices (20, 52) used during an operation, comprising a first control unit (12) for control of said devices, **characterized in that** a second control unit (14) is provided which is connected to the first control unit (12) for exchange of information, and the first control unit (12) is embodied as closed system with an embedded operating system for control of at least those devices (20; 22-26) which carry out safety-related functions, the first control unit (12) checks the second control unit (14), and the second control unit is embodied as open system with a non-embedded operating system for control of the remaining devices (52) which carry out non safety-related functions and the second control unit (14) checks the messages sent by the first control unit (12) on error messages and upon an error message displays it on a peripheral device, and that an interface unit (30) is provided, which is connected on the one-hand side with both control units (12, 14) and on the other hand side with peripheral devices (40) and connects one of both control units (12, 14) with the peripheral units (40) at a time.

2. System of claim 1, **characterized in that** the first control unit (12) and the safety-related devices (20) are interconnected via a bus system (28).

3. System of claim 1 or 2, **characterized in that** the non safety-related devices (52) and the second control unit (14) are interconnected via a further bus system (50).

4. System of claim 2 or 3, **characterized in that** both bus systems (28, 50) are different.

5. System of claim 5, **characterized in that** the interface unit (30) is controlled by the first control unit (12) via a control line (38).

6. System of claim 1 or 5, **characterized in that** the peripheral devices (40) include a monitor device (42) and/or an input device (44, 46), preferably a keyboard and a mouse.

7. System of claim 6, **characterized in that** the monitor device (42) is provided as a touch-screen also allowing an input.

8. System of claim 1, 5, 6 or 7, **characterized in that** the second control unit (14) comprises a receiving means to capture error messages from the first control unit (12) and to display them on one of the peripheral devices (40).

9. System of anyone of the preceding claims, **characterized in that** the safety-related devices (20) comprises endoscopic devices, preferably insufflators, pumps, light sources, video devices etc..

10. System of anyone of the preceding claims, **characterized in that** the non safety-related devices (52) may comprise picture archiving, OP-lighting, room lighting, telephone, air conditioning, pager, internet, hospital system, consumption parts, management systems, etc.

11. System of anyone of the preceding claims, **characterized in that** both control units (12, 14) are interconnected via an Ethernet bus (16).

12. System of anyone of the preceding claims, **characterized in that** the first control unit (12) comprises a voice control unit.

13. System of anyone of the preceding claims, **characterized in that** both control units (12, 14) are provided as a dual processor (CPU) computer unit.

14. System of claim 1, **characterized in that** the first control unit comprises a check means which cyclically checks the connection to the interface unit (30) and outputs an error message if a connection is not present.

15. Method for the central control of devices used during an operation, **characterized in that** the devices for carrying out safety-related functions are controlled by a first control unit (12) as closed system with an embedded operating system and the devices for carrying out non safety-related functions are controlled by a second control unit (14) as an open system with a non-embedded operating system, both control units (12, 14) communicate with each other, particularly the first control unit checking the second control unit for faults, an interface unit (30) is provided which is controlled by the first control unit (12) and dependent thereon forwards signals either from the first or the second control unit to a common peripheral device (40), and the first control unit (12) drives the interface unit (30) upon an error of the second control unit (14) such that the signals of the first control unit are forwarded to the peripheral devices (40).

16. Method of claim 15, **characterized in that** the interface unit (30) immediately forwards the signals of the first control unit (12) to the peripheral devices (40) if a safety-related function is to be carried out.

17. Method of claim 15, **characterized in that** after activating a non safety-related function the interface unit (30) forwards the signals of the second control unit (14) to the peripheral devices (40) only if the safety-related function is completed.

## Revendications

1. Système pour la commande centrale de dispositifs (20, 52), qui sont utilisés pendant une opération, comprenant une première unité de commande (12) pour la commande des dispositifs, **caractérisé en ce qu'**il est prévu une seconde unité de commande (14), qui est reliée à la première unité de commande (12) pour l'échange d'informations, et **en ce que** la première unité de commande (12) est équipée en tant que système fermé d'un système d'exploitation intégré et est conçue pour la commande au moins des dispositifs (20 ; 22-26) qui exécutent des fonctions importantes au niveau de la sécurité, **en ce que** la première unité de commande (12) contrôle la seconde unité de commande (14), et **en ce que** la seconde unité de commande (14) est équipée en tant que système ouvert d'un système d'exploitation non intégré et conçu pour la commande des autres dispositifs (52) qui n'exécutent pas des fonctions importantes au niveau de la sécurité, et **en ce que** la seconde unité de commande (14) contrôle les informations fournies par la première unité de commande (12) au niveau des messages d'erreurs et, dans le cas d'un message d'erreur, le présente sur un appareil périphérique, et **en ce qu'**il est prévue une unité d'interface (30) qui est reliée d'une part aux deux unités de commande (12, 14) et d'autre part à des appareils périphériques (40), et relie à chaque fois l'une des deux unités de commande (12, 14) aux appareils périphériques (40).

2. Système selon la revendication 1, **caractérisé en ce que** la première unité de commande (12) et les dispositifs (20) importants au niveau de la sécurité sont reliés entre eux au moyen d'un système de bus (28).

3. Système selon la revendication 1 ou 2, **caractérisé en ce que** les dispositifs (52) importants au niveau de la sécurité et la seconde unité de commande (14) sont reliés entre eux au moyen d'un autre système de bus (50).

4. Système selon les revendications 2 et 3, **caractérisé en ce que** les deux systèmes de bus (28, 50) sont différents.

5. Système selon la revendication 1, **caractérisé en ce que** le dispositif d'interface (30) est commandé au moyen d'une ligne de commande (38) par la première unité de commande (12).

6. Système selon la revendication 1 ou 5, **caractérisé en ce que** les appareils périphériques (40) comprennent un dispositif d'affichage (42) et/ou un dispositif d'entrée (44, 46), de préférence un clavier et une souris.

7. Système selon la revendication 6, **caractérisé en ce que** le dispositif d'affichage (42) est conçu comme écran tactile, de sorte que des entrées par ce moyen sont également possibles.

8. Système la revendication 1, 5, 6 ou 7, **caractérisé en ce que** la seconde unité de commande (14) présente un moyen de réception pour enregistrer des messages d'erreurs provenant de la première unité de commande (12) et les présenter sur l'un des appareils périphériques (40).

9. Système selon l'une quelconque des revendications précédentes, **caractérisé en ce que** les équipements (20) importants au niveau de la sécurité comprennent des appareils endoscopiques, de préférence des insuffleurs, des pompes, des sources lumineuses, des appareils vidéo, etc.

10. Système selon l'une quelconque des revendications précédentes, **caractérisé en ce que** les dispositifs (52) non importants au niveau de la sécurité peuvent comprendre l'archivage d'images, l'éclairage OP, l'éclairage de l'espace, le téléphone, la climatisation, le "Pager", l'Internet, le système hospitalier, les matériaux consommables, les systèmes de gestion, etc.

11. Système selon l'une quelconque des revendications précédentes, **caractérisé en ce que** les deux unités de commande (12, 14) sont reliées entre elles au moyen d'un bus Ethernet (16).

12. Système selon l'une quelconque des revendications précédentes, **caractérisé en ce que** la première unité de commande (12) présente une unité de commande vocale.

13. Système selon l'une quelconque des revendications précédentes, **caractérisé en ce que** les deux unités de commande (12, 14) sont réalisées sous la forme d'une unité de calculateur avec deux processeurs (CPU).

14. Système selon la revendication 1, **caractérisé en ce que** la première unité de commande présente un moyen d'essai qui contrôle de façon cyclique la liaison avec l'unité d'interface (30) et envoie un message d'erreur lorsqu'une liaison n'est pas présente.

15. Procédé pour le contrôle central de dispositifs qui sont utilisés pendant une opération, **caractérisé en ce que**
les dispositifs pour la mise en oeuvre de fonctions importantes au niveau de la sécurité sont commandés par une première unité de commande (12) en tant que système fermé avec système d'exploitation intégré et les dispositifs pour l'exécution de fonctions non importantes au niveau de la sécurité sont commandés par une seconde unité de commande (14) en tant que système ouvert avec système d'exploitation non intégré,
les deux unités de commande (12, 14) communiquent entre elles, sachant qu'en particulier la première unité de commande surveille la seconde unité de commande au niveau des erreurs,
une unité d'interface (30) est mise à disposition, laquelle est commandée par la première unité de commande (12) et transmet en fonction de cela des signaux soit de la première soit de la seconde unité de commande à des appareils périphériques (40) communs, et
la première unité de commande (12) active l'unité d'interface (30) dans le cas d'une erreur de la seconde unité de commande (14) de telle sorte que les signaux de la première unité de commande (12) sont transmis aux appareils périphériques (40).

16. Procédé selon la revendication 15, **caractérisé en ce que** l'unité d'interface (30) transmet immédiatement les signaux de la première unité de commande (12) aux appareils périphériques (40) lorsqu'une fonction importante au niveau de la sécurité doit être exécutée.

17. Procédé selon la revendication 15, **caractérisé en ce que**, après l'activation d'une fonction non importante au niveau de la sécurité, l'unité d'interface (30) ne transmet les signaux de la seconde unité de commande (14) aux appareils périphériques (40) qu'une fois que la fonction importante au niveau de la sécurité a été correctement exécutée.
